# EUROPÄISCHE PATENTSCHRIFT

(11) **EP 1 136 478 B1**
(45) Veröffentlichungstag und Bekanntmachung des Hinweises auf die Patenterteilung: **16.06.2004**
(21) Anmeldenummer: 01105734.6
(22) Anmeldetag: 08.03.2001
(51) Int. Cl.: C07D 319/06, C09K 19/58

(54) **Chirale 1,3-Dioxanverbindungen**
Chiral 1,3-dioxane compounds
Composés 1,3-dioxane chiraux

(30) Priorität: 20.03.2000 DE 10013507
(43) Veröffentlichungstag der Anmeldung: 26.09.2001
(73) Patentinhaber: BASF AKTIENGESELLSCHAFT, 67056 Ludwigshafen (DE)
(72) Erfinder: Prechtl, Frank, Dr., 60318 Frankfurt (DE); Haremza, Sylke, Dr., 69151 Neckargemünd (DE); Meyer, Frank, Dr., 69115 Heidelberg (DE); Parker, Robert, 68161 Mannheim (DE); Kürschner, Kathrin, Dr., 68199 Mannheim (DE); Vill, Volkmar, Dr., 20255 Hamburg (DE); Paul, Matthias, Dr., 22587 Hamburg (DE)

(56) Entgegenhaltungen:
- EP-A- 0 457 105
- EP-A- 0 541 081
- EP-A- 0 684 246
- EP-A- 0 774 452
- EP-A- 0 782 995
- WO-A-93/13093
- WO-A-97/00600
- WO-A-98/47979
- DE-A- 4 423 044
- DE-A- 19 625 441
- DE-A- 19 748 818
- DE-A- 19 835 730
- SZULC J ET AL: "THREE RING DIOXANES AS DOPANTS ENHANCING THE STABILITY OF THE SMECTIC C PHASE" LIQUID CRYSTALS, TAYLOR AND FRANCIS LTD, LONDON, GB, Bd. 14, Nr. 5, 1. Mai 1993 (1993-05-01), Seiten 1377-1387, XP000383398 ISSN: 0267-8292

## Beschreibung

Die Erfindung betrifft chirale 1,3-Dioxanverbindungen und deren Diastereomere und die Verwendung dieser Verbindungen als chirale Dotierstoffe für flüssigkristalline Systeme.

Weiter betrifft die Erfindung nicht polymerisierbare oder polymerisierbare flüssigkristalline Zusammensetzungen, welche mindestens eine erfindungsgemäße chirale 1,3-Dioxanverbindung enthalten, die Verwendung dieser nicht polymerisierbaren oder polymerisierbaren flüssigkristallinen Zusammensetzungen für die Herstellung optischer Bauelemente, die Verwendung der polymerisierbaren flüssigkristallinen Zusammensetzungen zum Bedrucken oder Beschichten von Substraten, zur Herstellung von Dispersionen und Emulsionen, Filmen oder Pigmenten sowie solche optischen Bauelemente, bedruckte oder beschichtete Substrate, Dispersionen und Emulsionen, Filme und Pigmente.

Zur Herstellung cholesterischer Flüssigkristallmischungen bedient man sich meist eines flüssigkristallinen (nematischen) Basismaterials und eines oder mehrerer optisch aktiver Dotierstoffe. Hierdurch lassen sich die optischen Eigenschaften der Mischung durch einfache Veränderung des Verhältnisses von Nemat zu Dotierstoff variieren. Um jedoch mögliche negative Einflüsse des Dotierstoffs auf die sonstigen Eigenschaften des nematischen Wirtsystems, wie z.B. Phasenverhalten und -breite, klein zu halten, sind besonders Dotierstoffe gefragt, welche bereits in kleinen Zugaben große Änderungen in den optischen Eigenschaften bewirken.

Chirale Dotierstoffe für flüssigkristalline Phasen sind in großer Zahl aus der wissenschaftlichen und Patentliteratur bekannt. Umso erstaunlicher ist es, dass chirale 1,3-Dioxanverbindungen als Dotierstoffe für flüssigkristalline Systeme offensichtlich noch nicht in Betracht gezogen wurden.

Die Aufgabe der vorliegenden Erfindung bestand nun darin, weitere chirale Verbindungen bereitzustellen, welche für die Herstellung von cholesterisch-flüssigkristallinen Zusammensetzungen geeignet sind, ein relativ hohes Verdrillungsvermögen besitzen und dementsprechend bereits in vergleichsweise kleinen Mengen großen Einfluss auf die optischen Eigenschaften des flüssigkristallinen Wirtssystems zeigen.

Diese Aufgabe wurde erfindungsgemäß durch die chiralen Verbindungen der allgemeinen Formel I 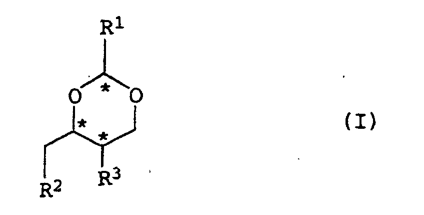 und deren Diastereomere gelöst.

Hierbei bedeuten
- R¹: eine Gruppierung [P-Y¹-(A¹)ₘ-Y²-]_{q}M-Y³-(A²)ₙ-Y⁴- und
- R² und R³: unabhängig voneinander und unabhängig von R¹ Gruppierungen [P-Y¹-(A¹)ₘ-Y²-]_{q}M-Y³-(A²)ₙ-Y⁴'-
mit
- A¹, A²: Spacer mit ein bis 30 Kohlenstoffatomen,
- M: mesogene Gruppe,
- Y¹, Y², Y³, Y⁴: chemische Einfachbindung, -O-, -S-, -CO- -CO-O-, -O-CO-, -CO-N(R)-, -(R)N-CO-, -O-CO-O-, -O-CO-N(R)-, -(R)N-CO-O- oder -(R)N-CO-N(R)-,
- Y⁴': -O-, -O-CO-, -O-CO-O- oder -O-CO-N(R)-,
- R: Wasserstoff oder C₁-C₄-Alkyl,
- P: Wasserstoff, C₁-C₁₂-Alkyl, eine polymerisierbare oder zur Polymerisation geeignete Gruppe oder ein Rest, welcher eine polymerisierbare oder zur Polymerisation geeignete Gruppe trägt,
- m,n: Werte von 0 oder 1 und
- q: Werte von 1, 2 oder 3,
wobei die Reste A¹, A², Y¹, Y², Y³, Y⁴, Y⁴', M und P und die Indices m, n und q der Reste R¹ bis R³ gleich oder verschieden sein können und die Reste R² und R³ über das Sauerstoffatom des Restes Y⁴' an das 1,3-Dioxangerüst angebunden sind, mit der Maßgabe, dass für den Fall, dass einer oder beide der Indices m und n gleich 0 sind, mindestens einer der jeweils von A benachbarten Reste Y eine chemische Bindung bedeutet.

Als Spacer A¹ und A² kommen alle dem Fachmann für diesen Zweck bekannten Gruppen in Betracht. Die Spacer enthalten in der Regel ein bis 30, vorzugsweise ein bis 12, besonders bevorzugt ein bis sechs Kohlenstoffatome und bestehen aus vorwiegend linearen aliphatischen Gruppen. Sie können in der Kette, z.B. durch nicht benachbarte Sauerstoff- oder Schwefelatome oder Imino- oder Alkyliminogruppen wie beispielsweise Methyliminogruppen, unterbrochen sein. Als Substituenten für die Spacerkette kommen dabei noch Fluor, Chlor, Brom, Cyan, Methyl und Ethyl in Betracht.

Repräsentative Spacer sind beispielsweise:
- (CH₂)ᵤ-, -(CH₂CH₂O)ᵥCH₂CH₂-, -CH₂CH₂SCH₂CH₂-, -CH₂CH₂NHCH₂CH₂-, 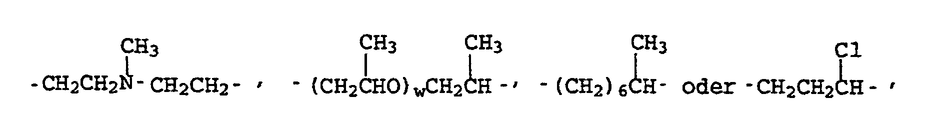 wobei u für 1 bis 30, vorzugsweise 1 bis 12, v für 1 bis 14, vorzugsweise 1 bis 5, und w für 1 bis 9, vorzugsweise 1 bis 3, steht.

Bevorzugte Spacer sind Ethylen, Propylen, n-Butylen, n-Pentylen und n-Hexylen.

Als mesogene Gruppen M können alle dem Fachmann als solche bekannte Gruppen dienen.

Insbesondere kommen mesogene Gruppen der Formel Ia

(-T-Y⁵)ᵣ-T- Ia

in Betracht, worin die Variablen bedeuten
- T: zweiwertige gesättigte oder ungesättigte carbo- oder heterocyclische Reste
- Y⁵: -chemische Einfachbindung, -O-, -S-, -CO-, -CO-O-, -O-CO-, - CO-N(R)-, -(R)N-CO-,-O-CO-O-, -O-CO-N(R)-, -(R)N-CO-O- oder - (R)N-CO-N(R)- und
- r: Werte von 0, 1, 2 oder 3, wobei für r > 0 sowohl die Reste T als auch die Gruppen Y⁵ untereinander gleich oder verschieden sein können.

Anmerkung:Die Definition des Restes T als zweiwertig muss dahingehend konkretisiert werden, dass diese Zweiwertigkeit natürlich nur im Bezug auf die Anknüpfung eines betrachteten Restes T an den oder die benachbarten Reste T, eine der q Einheiten P-Y¹-(A¹)ₘ-Y²- und/oder die Einheit -Y³-(A²)ₙ-Y⁴- bzw. -Y³-(A²)ₙ-Y⁴'- zu sehen ist.

Sind an die mesogene Gruppe M, welche als solche gemäß ihrer Definition in Formel I (q+1)-wertig ist, z.B. zwei oder drei Einheiten P-Y¹-(A¹)ₘ-Y²- angebunden (q gleich zwei oder drei), so erhöht sich natürlich für mindestens einen betrachteten Rest T die Wertigkeit auf drei oder gegebenenfalls sogar vier.

Exemplarisch sei dies für q und r jeweils gleich 3 für zwei der möglichen Isomeren, nämlich 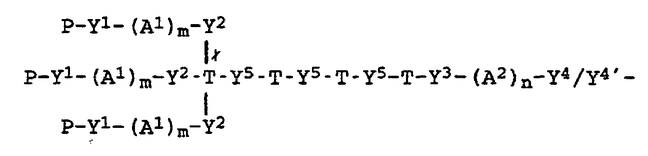 oder auch 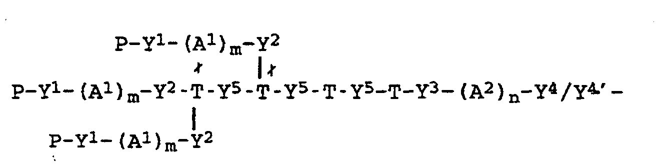 veranschaulicht.

In ersterem Fall ist der betrachtete Rest T (mit 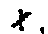 markiert) zweiwertig im Bezug auf die Anbindung einer Einheit P-Y¹-(A¹)ₘ-Y²- und eines benachbarten Restes T, insgesamt jedoch vierwertig, da noch zwei weitere Einheiten P-Y¹-(A¹)ₘ-Y²an ihn gebunden sind. In letzterem Fall sind die beiden betrachten Reste T zweiwertig im Bezug auf die Anbindung einer Einheit P-Y¹-(A¹)ₘ-Y²- und eines benachbarten Restes T bzw. zweier benachbarter Reste T, durch Anbindung je einer weiteren Einheit P-Y¹-(A¹)ₘ-Y²- ergibt sich für beide betrachteten Reste T jedoch eine Wertigkeit von drei.

Weiter soll hier wie auch im Rahmen der gesamten Anmeldung mindestens eine der q Einheiten P-Y¹-(A¹)ₘ-Y²- terminal an die mesogene Gruppe M gemäß den Formeln I oder Ia angebunden sein.

Die Reste T können durch Fluor, Chlor, Brom, Cyan, Hydroxy, Formyl, Nitro, C₁-C₂₀-Alkyl, C₁-C₂₀-Alkoxy, C₁-C₂₀-Alkoxycarbonyl, C₁-C₂₀-Monoalkylaminocarbonyl, C₁-C₂₀-Alkylcarbonyl, C₁-C₂₀-Alkylcarbonyloxy oder C₁-C₂₀-Alkylcarbonylamino substituierte Ringsysteme sein. Bevorzugte Reste T sind: 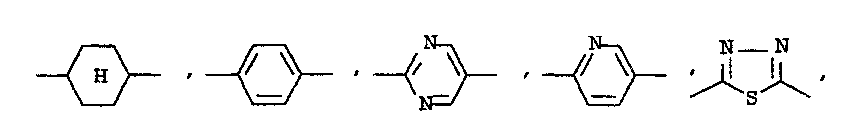 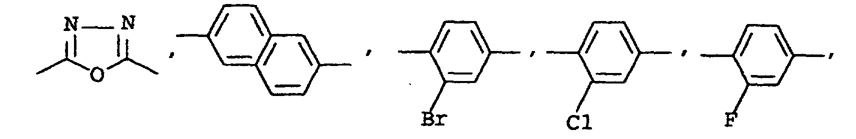 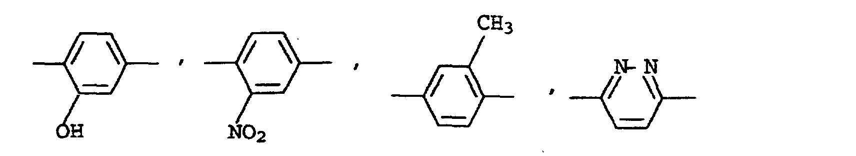 und

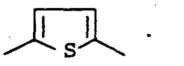

Als mesogene Gruppen M für R¹, R² und R³ kommen in Frage beispielsweise: 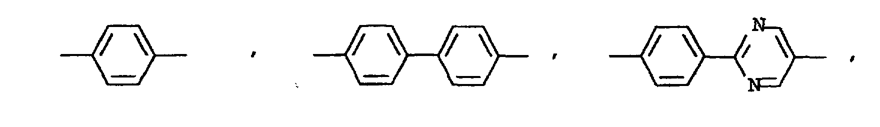 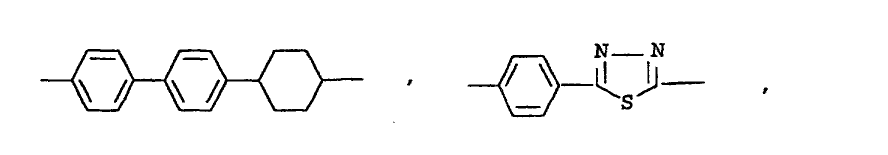 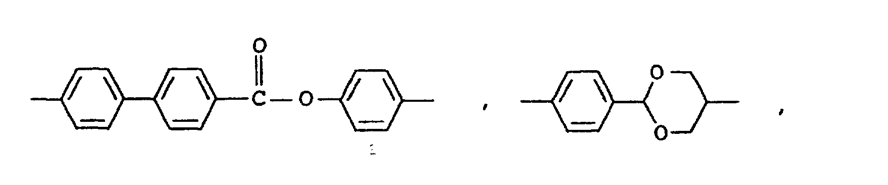 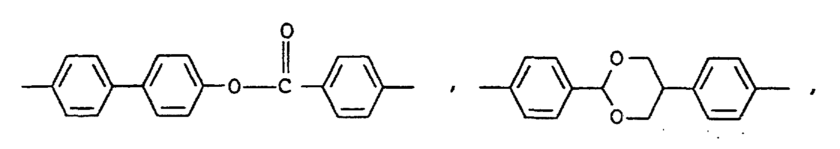 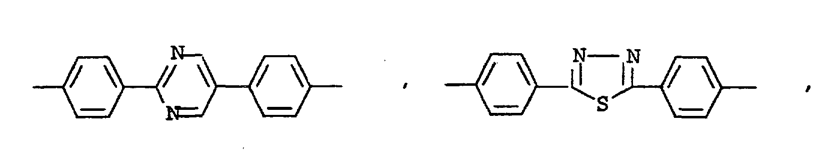 oder

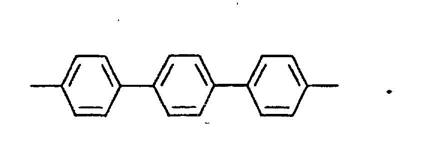

Weitere mögliche mesogene Gruppen M gehorchen den folgenden Formeln:

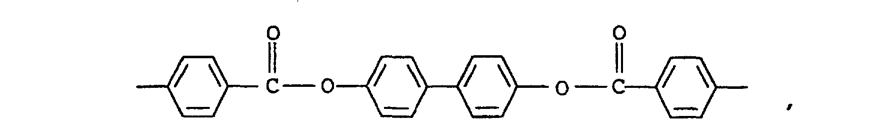

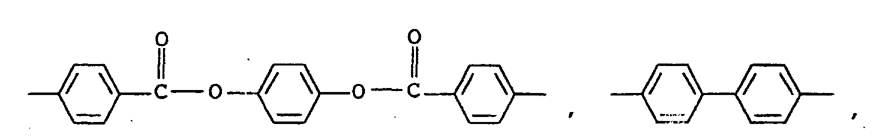

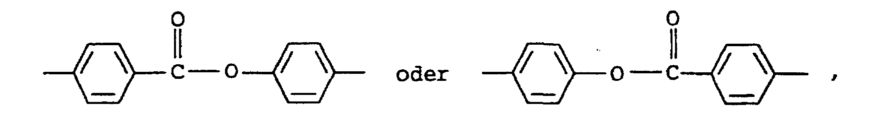

Die zuvor gezeigten (unsubstituierten) mesogenen Gruppen können natürlich entsprechend der oben gegebenen Beispiele für mögliche Reste T noch durch Fluor, Chlor, Brom, Cyan, Hydroxy, Formyl, Nitro, C₁-C₂₀-Alkyl, C₁-C₂₀-Alkoxy, C₁-C₂₀-Alkoxycarbonyl, C₁-C₂₀-Monoalkylaminocarbonyl, C₁-C₂₀-Alkylcarbonyl, C₁-C₂₀-Alkylcarbonyloxy oder C₁-C₂₀-Alkylcarbonylamino substituiert sein. Bevorzugte Substituenten sind vor allem kurzkettige aliphatische Reste wie Methyl, Ethyl, n-Propyl, i-Propyl, n-Butyl, i-Butyl, sek.-Butyl, tert.-Butyl sowie Alkoxy-, Alkoxycarbonyl-, Alkylcarbonyl-, Alkylcarbonyloxy-, Alkylcarbonylamino- und Monoalkylaminocarbonylreste, die diese Alkylgruppen enthalten.

Bevorzugte 1,3-Dioxanverbindungen sind hierbei solche, in welchen in der mesogenen Gruppe der Formel Ia für den Rest R¹ der Index r den Wert 0 und für die Reste R² und R³ unabhängig voneinander die Werte 0 oder 1 annimmt. Vor allem sind hier für R¹ als mesogene Gruppen 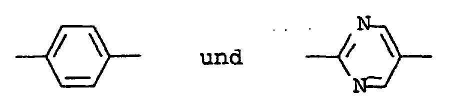 und für R² und R³: 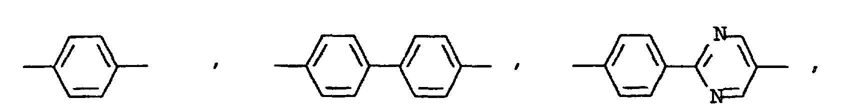 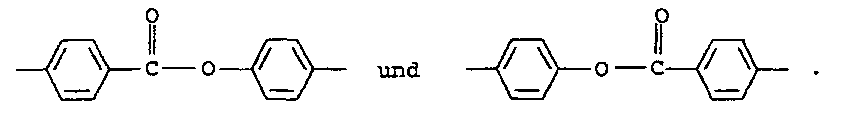 zu nennen. Darüber hinaus können diese mesogenen Gruppen, wie zuvor erwähnt, substituiert sein.

Ferner ist es auch möglich, einen oder mehrere der mesogenen Reste M ohne Spacer A¹ direkt mit den entsprechenden Gruppen P zu verknüpfen. In diesen Fällen nehmen die Indices m bzw. n den Wert 0 an und Y¹/Y² stehen gemeinsam für eine chemische Einfachbindung.

Als C₁-C₁₂-Alkyl für P sind verzweigte oder unverzweigte C₁-C₁₂-Alkylketten zu nennen, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl; n-Undecyl und n-Dodecyl.

Bevorzugtes Alkyl für P sind die verzweigten oder unverzweigten C₁-C₆-Alkylketten, wie etwa Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl.

Als polymerisierbare oder zur Polymerisation geeignete Gruppe oder als Rest, welcher eine polymerisierbare oder zur Polymerisation geeignete Gruppe trägt (nachfolgend werden solche Gruppen oder Reste auch einfach als "reaktive Reste" bezeichnet), kommen für P in Frage: 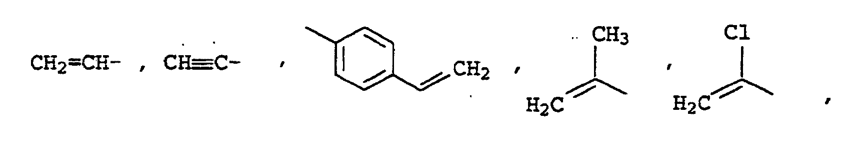 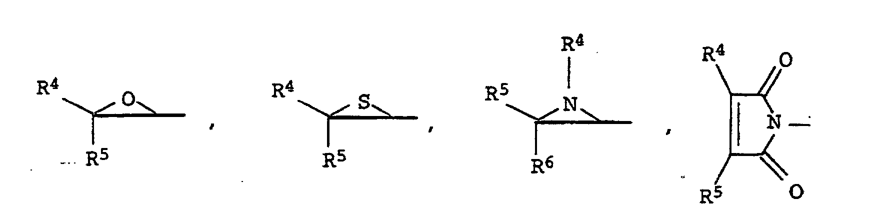 -N=C=O, -N=C=S, -O-C≡N, -COOH, -OH oder NH₂
wobei die Reste R⁴ bis R⁶ gleich oder verschieden sein können und Wasserstoff oder C₁-C₄-Alkyl, wie Methyl, Ethyl, n-Propyl, isoPropyl, n-Butyl, iso-Butyl, sec.-Butyl oder tert.-Butyl bedeuten.

Von den polymerisierbaren Gruppen können die Cyanate spontan zu Cyanuraten trimerisieren. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen, wie beispielsweise Styrol oder Verbindungen, welche Styrolstrukturelemente enthalten.

Die komplementären reaktiven Reste können dabei, zusammen mit den ihnen entsprechenden reaktiven Resten, in ein und derselben erfindungsgemäßen 1,3-Dioxanverbindung zugegen sein (so dass diese Verbindung potenziell auch mit sich selbst polymerisieren kann) oder in einer weiteren erfindungsgemäßen 1,3-Dioxanverbindung. Diese komplementären reaktive Reste können sich aber auch, zusammen mit den entsprechenden reaktiven Resten, in ein und derselben (Hilfs-)Verbindungen oder in weiteren solchen (Hilfs-)Verbindungen befinden.

Als polymerisierbare Gruppe ist insbesondere der Acrylat-, Methacrylat- sowie der Vinylrest hervorzuheben.

Bevorzugt sind weiter Verbindungen der Formeln I, in welchen im Rest R¹ die Einheit P-Y¹-(A¹)ₘ-Y²- Wasserstoff bedeutet, d.h. P steht für Wasserstoff, Y¹ bzw. Y² jeweils für eine chemische Einfachbindung und m für 0, und in mindestens einer der Einheiten P-Y¹-(A¹)ₘ-Y²- der beiden Reste R² und R³ der Index m verschieden von 0 ist. Das bedeutet, dass im Rahmen dieser Bevorzugung mindestens einer der letzteren Reste eine über Spacer A¹ und Y¹/Y² an die mesogene Gruppe M angebundene Gruppe P besitzt und sich im Falle von R¹ die Gruppierung [P-Y¹-(A¹)ₘ-Y²-]_{q}M-Y³- -(A²)ₙ-Y⁴- auf [H-]_{q}M-Y³-(A²)ₙ-Y⁴- oder, unter Berücksichtigung von Formel Ia, auf [H-]_{q}(-T-Y⁵)ᵣ-T-Y³-(A²)ₙ-Y⁴- reduziert.

Im Sinne der beiden bereits weiter oben in der Anmerkung exemplarisch angesprochenen Fälle ergeben sich damit 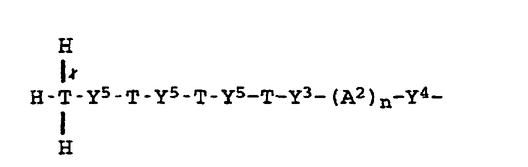 und

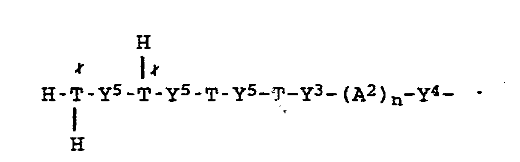

Dies ist natürlich gleichbedeutend damit, dass die mesogene Gruppe M im Rest R¹ nicht weiter mit Einheiten P-Y¹-(A¹)ₘ-Y²- substituiert ist.

Bevorzugt sind zudem erfindungsgemäße Verbindungen, in welchen q in den beiden Resten R² und R³ den Wert 1 annimmt, d.h jede mesogene Gruppe M der Reste R2 und R3 ist nur mit einer Einheit P-Y¹-(A¹)ₘ-Y²- substituiert, welche sich dann entsprechend den Ausführungen in oben gemachter Anmerkung terminal gebunden an der mesogenen Gruppe M befindet, d.h. entsprechend den beiden oben gegebenen Beispielen ist zu schreiben 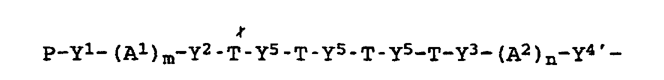 und 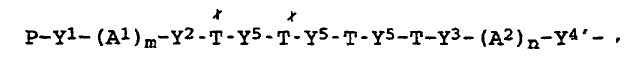 was natürlich in diesem Fall gleichbedeutend ist.

Besondere Bevorzugung im Rahmen der vorliegenden Erfindung finden chirale Verbindungen basierend auf dem Diastereomer der Formel I', 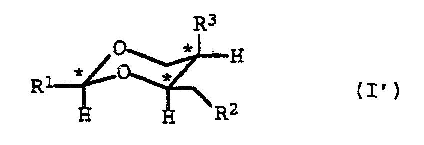 worin R¹, R² und R³ die für Formel I angegebene Bedeutung besitzen. Die zuvor genannten Bevorzugung gelten sinngemäß auch für die Verbindungen der Formel I'.

Erfindungsgemäß finden die Verbindungen der Formel I und deren bevorzugte Ausführungsformen Verwendung als chirale Dotierstoffe für flüssigkristalline Systeme. Der Begriff "flüssigkristalline Systeme" sei hierbei nicht nur auf Systeme beschränkt, in welchen ein oder mehrere Bestandteile bereits per se (im interessierenden Temperaturbereich) flüssigkristalline Eigenschaften besitzen und diese auch im System vorhanden sind, vielmehr sind hierunter auch solche Systeme zu verstehen, in welchen sich erst durch Mischen der Komponenten oder auch erst durch Zumischen der chiralen erfindungsgemäßen Verbindunge(en) flüssigkristallines Verhalten manifestiert (z.B. lyotrope Systeme). Anzumerken sei bier ferner, dass die erfindungsgemäßen Verbindungen nicht bereits selbst flüssigkristallines Verhalten aufweisen müssen.

Beansprucht werden weiter flüssigkristalline und polymerisierbare flüssigkristalline Zusammensetzungen, welche mindestens eine chirale Verbindung der Formel I oder einer bevorzugten Ausführungsform enthalten.

Unter den flüssigkristallinen Zusammensetzungen sind hier insbesondere nicht polymerisierbare Zusammensetzungen gemeint, welche unter üblichen Bedingungen nicht zur Ausbildung von Polymerisations- oder Kondensationsprodukten befähigt sind. Diese Zusammensetzungen lassen sich etwa durch Vermischen geeigneter, kommerziell erhältlicher flüssigkristalliner Materialien, wie sie zum Beispiel für aktive LC-Schichten in der Displaytechnik Verwendung finden, mit einer oder mehreren der erfindungsgemäßen Verbindungen herstellen. In letzteren entspricht P in Formel I dementsprechend Wasserstoff oder C₁-C₁₂-Alkyl.

Erfindungsgemäß wird die Verwendung dieser (nicht polymerisierbaren) flüssigkristallinen Zusammensetzungen für die Herstellung optischer Bauelemente, wie z.B. LCDs, beansprucht. Solchermaßen erhaltene optische Bauelemente werden erfindungsgemäß ebenfalls beansprucht.

Weiterer Gegenstand der vorliegenden Erfindung sind polymerisierbare flüssigkristalline Zusammensetzungen. Hierunter sind insbesondere solche Zusammensetzungen zu verstehen, in welchen mindestens einer der Bestandteile unter üblichen Bedingungen zur Ausbildung von Polymerisations- oder Kondensationsprodukten befähigt ist.

Je nach Anzahl der reaktiven Reste in den Bestandteilen dieser Zusammensetzungen, lässt sich der gewünschte Polymerisations-, Vernetzungs- und/oder Kondensationsgrad nach erfolgter Polymerisation oder Kondensation einstellen. Die erfindungsgemäßen Verbindungen der Formel I besitzen in solchen Zusammensetzungen mindestens eine, vorzugsweise zwei reaktive Reste P, welche insbesondere an die Reste R² und R³ gebunden sind. Diese Zusammensetzungen lassen sich leicht durch Vermischen geeigneter polymerisierbarer, flüssigkristalliner Materialien mit einer oder mehreren der erfindungsgemäßen Verbindungen herstellen. Geeignete polymerisierbare, flüssigkristalline Verbindungen sind beispielsweise in den WO-Schriften 95/22586, 95/24454, 95/24455, 96/04351, 96/24647, 97/00600, 97/34862 und 98/47979 sowie der deutschen Offenlegungsschrift 198 35 730 beschrieben und entsprechen im Wesentlichen dem schematischen Aufbau P-Y-A-Y-M-Y-A-Y-P, worin die Variablen P, Y, A und M analoge Bedeutungen wie die Variablen P, Y¹ bis Y⁴, A¹, A² und M in Formel I besitzen.

Erfindungsgemäß wird die Verwendung dieser polymerisierbaren flüssigkristallinen Zusammensetzungen für die Herstellung optischer Bauelemente, wie z.B. Polarisatoren oder Filtern, beansprucht.

Gegenstand der vorliegenden Erfindung sind zudem solche optischen Bauelemente, welche unter Verwendung dieser erfindungsgemäßen polymerisierbaren flüssigkristallinen Zusammensetzungen erhalten wurden.

Erfindungsgemäß finden die beanspruchten polymerisierbaren flüssigkristallinen Zusammensetzungen Verwendung zum Bedrucken oder Beschichten von Substraten. Hierbei können diese Zusammensetzungen noch weitere Zusätze enthalten. Als solche kommen in Frage Zusätze ausgewählt aus der Gruppe bestehend aus Photoinitiatoren, Reaktivverdünnern und Verdünnungsmitteln, Zusätze ausgewählt aus der Gruppe bestehend aus Entschäumern und Entlüftern, Gleit- und Verlaufshilfsmitteln, thermisch härtenden oder strahlenhärtenden Hilf smitteln, Substratnetzhilfsmitteln, Netz- und Dispergierhilfsmitteln, Hydrophobierungsmitteln, Haftvermittlern und Hilfsmitteln zur Verbesserung der Kratzfestigkeit, Zusätze ausgewählt aus der Gruppe bestehend aus Farbstoffen und Pigmenten und Zusätze ausgewählt aus der Gruppe der Licht-, Hitze- und/oder Oxidationsstabilisatoren.

Die chemisch-physikalische Natur dieser Zusätze wird ausführlich in der älteren deutschen Anmeldung 199 05 394.4 gewürdigt. Weiter sind in dieser Schrift flüssigkristalline Stoffgemenge beschrieben, als welche auch die erfindungsgemäßen polymerisierbaren flüssigkristallinen Zusammensetzungen, gegebenenfalls in Mischung mit den zuvor erwähnten Zusätzen, anzusprechen sind. Die in vorliegender Anmeldung beanspruchten polymerisierbaren flüssigkristallinen Zusammensetzungen können, gegebenenfalls in Mischung mit besagten Zusätzen, dementsprechend wie in der älteren deutschen Schrift 199 05 394.4 ausgeführt, als Bedruckungs- und Beschichtungsmittel für Substrate eingesetzt werden.

Im Rahmen der vorliegenden Erfindung werden weiter bedruckte oder beschichtete Substrate beansprucht, welche unter Verwendung der erfindungsgemäßen polymerisierbaren Zusammensetzungen, gegebenenfalls in Mischung mit den zuvor erwähnten Zusätzen, hergestellt worden sind.

Als solche Substrate kommen neben Papier- und Kartonageprodukten, beispielsweise für Tragetaschen, Zeitschriften, Broschüren, Geschenkverpackungen und Verpackungsmaterialien für Gebrauchs-, Genuss- und Luxusgüter, auch Folien, etwa für dekorative und nichtdekorative Verpackungszwecke, sowie Textilien jedweder Art und Leder in Frage.

Weitere Substrate sind aber auch Güter der (Unterhaltungs-)Elektronik, wie etwa MC-, MD-, DVD- und Videorecorder, Fernseher, Radios, Telefone/Handys usw. und EDV-Geräte, Güter aus dem Freizeit-, Sport-, Haushalts- und Spielsektor, wie etwa Fahrräder, Kinderfahrzeuge, Skier, Snow- und Surfboards, Inline-Skater und Roll- und Schlittschuhe sowie Haushaltsgeräte. Darüber hinaus sind unter solchen Substraten beispielsweise auch Schreibutensilien und Brillengestelle zu verstehen.

Weitere Substrate sind aber auch im Bausektor anzutreffende Oberflächen, wie Gebäudewände oder auch Fensterscheiben. In letzterem Fall kann neben einem dekorativen auch ein funktioneller Effekt gewünscht sein. So ist es möglich, Mehrfachschichten auf dem Fenstermaterial zu erzeugen, deren einzelne Schichten verschiedene chemisch-physikalische Eigenschaften besitzen. Werden etwa einzelne Schichten der polymerisierbaren flüssigkristallinen Zusammensetzungen entgegengesetzter Verdrillung (durch Verwendung des einen Enantiomeren sowie seines optischen Antipoden als Dotierstoff gemäß vorliegender Erfindung) oder einzelne Schichten von vernetzten cholesterisch flüssigkristallinen Zusammensetzungen gleicher Gangrichtung aber jeweils unterschiedlicher Ganghöhe und somit unterschiedlicher Reflektionseigenschaften (durch Verwendung unterschiedlicher Konzentrationen an Dotierstoff gemäß vorliegender Erfindung) aufgebracht, so können gezielt bestimmte Wellenlängen oder Wellenlängenbereiche des Lichtspektrums reflektiert werden. Hierdurch ist beispielsweise eine IR- oder UV-reflektierende Fensterbeschichtung möglich. Zu diesem Aspekt der erfindungsgemäßen Zusammensetzungen, speziell Wärmeisolationsbeschichtungen, sei auch auf die deutsche Offenlegungsschrift 197 45 647 verwiesen.

Beansprucht wird im Rahmen der vorliegenden Erfindung auch die Verwendung der erfindungsgemäßen polymerisierbaren flüssigkristallinen Zusammensetzungen zur Herstellung von Dispersionen und Emulsionen, welche bevorzugt auf Wasser basieren. Zur Herstellung solcher Dispersionen und Emulsionen sei auf die Schriften WO 96/02597 und WO 98/47979 verwiesen, in welchen die Herstellung von Dispersionen und Emulsionen unter Verwendung von flüssigkristallinen Materialien beschrieben ist.

Gegenstand der vorliegenden Erfindung sind mithin solche Dispersionen und Emulsionen, welche unter Verwendung der erfindungsgemäßen polymerisierbaren flüssigkristallinen Zusammensetzungen hergestellt wurde. Diese Dispersionen und Emulsionen können ebenfalls zum Bedrucken und Beschichten von Substraten, wie sie beispielhaft zuvor bereits beschrieben wurden, verwendet werden.

Die Verwendung der erfindungsgemäßen polymerisierbaren flüssigkristallinen Zusammensetzungen zur Herstellung von Filmen ist weiterer Gegenstand der vorliegenden Erfindung. Unter solchen Filmen sind insbesondere selbsttragende Schichten zu verstehen, wie sie durch Polymerisation der Zusammensetzungen erhalten werden. Diese Filme können sich auf Substraten bzw. Unterlagen befinden, welche so beschaffen sind, dass deren leichte Ablösung und Übertragung auf andere Substrate oder Unterlagen zum permanenten Verbleib durch geeignete Maßnahmen möglich ist. Solche Filme sind beispielsweise im Bereich der Folienbeschichtung und in Kaschierverfahren verwendbar.

Gegenstand der vorliegenden Erfindung sind dementsprechend auch solche Filme, welche unter Verwendung der erfindungsgemäßen polymerisierbaren flüssigkristallinen Zusammensetzungen hergestellt worden sind.

Weiter wird die Verwendung der erfindungsgemäßen polymerisierbaren flüssigkristallinen Zusammensetzungen zur Herstellung von Pigmenten beansprucht.

Die Herstellung solcher Pigmente ist bekannt und beispielsweise ausführlich in der Schrift WO 99/11733 beschrieben. Darüber hinaus lassen sich aber auch in Form und Größe voreingestellte Pigmente unter Verwendung von Drucktechniken oder mit Hilfe von Netzen, in deren Zwischenräumen sich die polymerisierbare Zusammensetzung befindet, herstellen. Der nachfolgenden Polymerisation oder Kondensation der flüssigkristallinen Zusammensetzung schließt sich hierbei das Ab- bzw. Herauslösen vom Substrat bzw. aus dem Netz an. Diese Vorgehensweisen sind in den Schriften WO 96/02597, WO 97/27251, WO 97/27252 und EP 0 931 110 ausführlich beschrieben.

Die polymerisierbaren flüssigkristallinen Zusammensetzungen können mit Hilfe ihrer reaktiven Gruppen und abhängig von deren chemischer Natur durch Kondensation oder radikalische oder ionische Polymerisationsverfahren, welche durch photochemische Reaktionen gestartet werden können, in Polymere mit eingefrorener flüssigkristalliner Ordnungsstruktur überführt werden.

Diese Pigmente können einschichtig (homogen) sein oder einen Mehrschichtaufbau aufweisen. Letztere Pigmente sind jedoch üblicherweise nur herstellbar, wenn Beschichtungsverfahren zur Anwendung kommen, in welchen sukzessive mehrere Schichten übereinander erzeugt und abschließend einer mechanischen Zerkleinerung unterworfen werden.

Gegenstand der vorliegenden Erfindung sind somit auch aus solchen erfindungsgemäßen polymerisierbaren flüssigkristallinen Zusammensetzungen hergestellte Pigmente.

### Beispiel 1a: Herstellung von 4,6-O-Benzyliden-D-galactopyranose

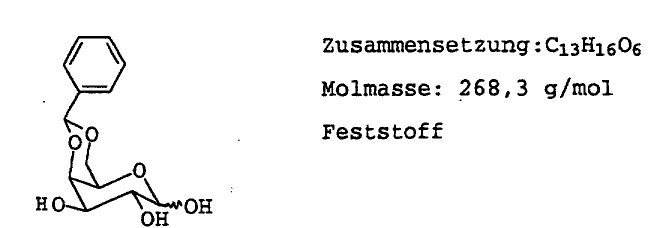

Eine Mischung aus 10,0 g D-Galactose (55,5 mmol), 9,1 ml Benzaldehyddimethylacetal (61,0 mmol) und 12 mg p-Toluolsulfonsäure in 40 ml Dimethylformamid wird bei 60°C intensiv gerührt, wobei man zur Entfernung des entstehenden Methanols einen konstanten Druck von 140 mbar einstellt. Nach etwa 25 min ist die Galactose komplett gelöst. Man unterbricht die Reaktion durch Abkühlen und Zugabe von 0,3 ml Triethylamin. Der Ansatz wird unter vermindertem Druck eingeengt und säulenchromatographisch gereinigt (Laufmittel: Ethylacetat + 0,1 Gew.-% Triethylamin). Ausbeute: 7,7 g (52% d. Th. )

### Beispiel 1b: Herstellung von 2,4-O-Benzyliden-D-threose

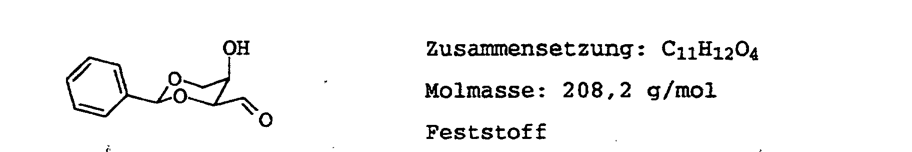

Zu einer Lösung von 12,60 g Natriummetaperiodat (60 mmol) und 2,4 g Natriumhydrogencarbonat (30 mmol) in 200 ml Wasser werden 6,7 g 4,6-O-Benzyliden-D-galactopyranose (25 mmol) gegeben. Nach 0,5 h wird der Ansatz unter vermindertem Druck eingeengt, der erhaltene Rückstand mit 200 ml Ethanol versetzt, erneut eingeengt und fünfmal mit je 200 ml warmen Ethylacetat extrahiert. Die vereinigten organischen Phasen werden mit Wasser gewaschen, über Natriumsulfat getrocknet und schließlich bei vermindertem Druck eingeengt. Das erhaltene Produkt wird sofort weiterverarbeitet. Ausbeute: 3,56 g (68% d.Th.)

### Beispiel 1c: Herstellung von 2,4-O-Benzyliden-D-threitol

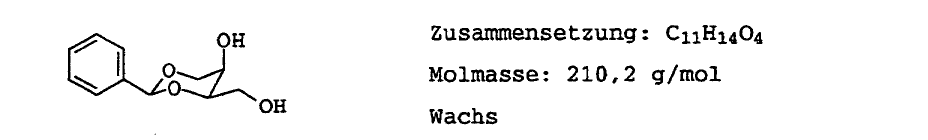

Zu einer Lösung von 3,56 g 2,4-O-Benzyliden-D-threose (17,1 mmol) in 75 ml Ethanol wird bei Raumtempertatur eine Lösung von 726 mg Natriumborhydrid (19,2 mmol) in 7,5 ml Wasser gegeben. Nach 2 h wird die Lösung unter vermindertem Druck eingeengt, der Rückstand in 100 ml Ethylacetat aufgenommen, zweimal mit 10 ml 10%iger Natriumsulfatlösung gewaschen, über Natriumsulfat getrocknet und schließlich unter vermindertem Druck eingeengt. Ausbeute: 3,06 g (85% d.Th.)

1H-NMR (400 MHz, CDCl₃): δ = 7,51 (mc, 2H, H_{Ar}-2, H_{Ar}-6), 7,38 (mc, 3H, H_{Ar}-3, H_{Ar}-4, H_{Ar}-5), 5,60 (s, 1H, H-2), 4,22 (dd, 1H, H-6a), 4,07 (dd, 1H, H-6b), 4,02 (ddd, 1H, H-4), 3,92 (dd, 1H, C***H***_{***A***}H_{B}OH), 3,83 (dd, 1H, CH_{A}***H***_{***B***}OH), 3,68 (ddd, 1H, H-5).

3J_{H-4, H-5} = 1,3, 3J_{H-4, H-A} = 6,7, 3J_{H-4, H-B} = 5,2, 3J_{H-5, H-6a} = 1,7, 3J_{H-5, H-6b} = 1,3, 2J_{H-A, H-B} = 11,6, 2J_{H-6a, H-6b} = 12,0 Hz.

13C-NMR (100 MHz, CDCl₃): δ = 137,52 (C_{Ar}-1), 129,24 (C_{Ar}-4), 128,48 (C_{Ar}-3, C_{Ar}-5), 126,02 (C_{Ar}-2, C_{Ar}-6), 101,57 (OCHO), 79,60 (C-4), 72,59 (C-6), 64,55 (C-5), 62,89 (CH₂OH).

### Beispiel 2: Herstellung von 4-Hexoxybenzoyl-4'-oxybenzoesäure

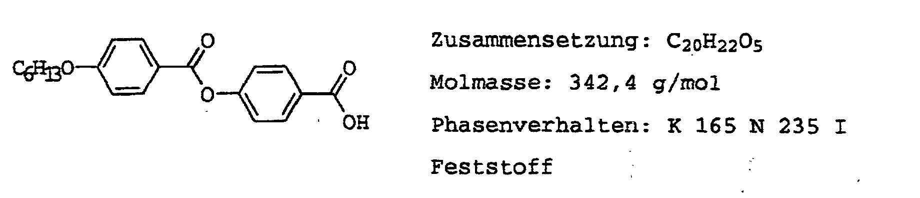

Zu einer Lösung von 6,90 g 4-Hydroxybenzoesäure (50 mmol) in 100 ml 1N Natronlauge wird bei 0°C unter Rühren eine Lösung von 11,1 ml 4-Hexyloxybenzoesäurechlorid (50 mmol) in 50 ml Aceton gegeben. Die milchig-trübe Lösung wird mit 6N Salzsäure auf pH 2-3 eingestellt und filtriert. Man löst den erhaltenen Feststoff in Dioxan und erhitzt für 4 h unter Rückfluß. Die Lösung wird schließlich unter vermindertem Druck eingeengt und der erhaltene Rückstand aus Aceton umkristallisiert. Ausbeute: 3,08 g (18% d.Th.)

1H-NMR (400 MHz, CDCl₃): δ = 8,20 (d, 2H, H_{Ar}-2', H_{Ar}-6'), 8,18 (d, 2H, H_{Ar}-2, H_{Ar}-6), 7,34 (d, 2H, H_{Ar}-3', H_{Ar}-5'), 6,98 (d, 2H, H_{Ar}-3, H_{Ar}-5), 4,05 (t, 2H, OC***H***_{***2***}C₅H₁₁), 1,83 (mc, 2H, OCH₂C***H***_{***2***}C₄H₉), 1,53-1,30 (m, 6H, 3xCH₂), 0,92 (t, 3H, CH₃).

3J_{Ar} = 9,1, 3J_{OCH2} = 6,6, 3J_{CH2CH3} = 7,1 Hz.

13C-NMR (100 MHz, CDCl₃): δ = 170,92 (COOH), 164,34, 163,83 (C_{Ar}-4, COO), 155,55 (C_{Ar}-4'), 132,43 (C_{Ar}-2', C_{Ar}-6'), 131, 89 (C_{Ar}-2, C_{Ar}-6), 126,62 (C_{Ar}-1'), 122,01(C_{Ar}-3', C_{Ar}-5'), 120,96 (C_{Ar}-1), 114,28 (C_{Ar}-3, C_{Ar}-5), 68,40 (OCH₂), 31,70, 29,47, 26,08, 23,01 (CH₂), 14,45 (CH₃).

### Beispiel 3: Herstellung von (2S,4R,5R)-4,5-Di-O-(4-(p-hexyloxybenzyoyloxy)benzoyl)-2-phenyl-1,3-dioxan

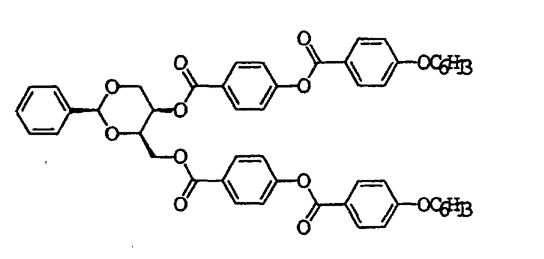

Zusammensetzung: C₅₁H₅₄O₁₂, Molmasse: 859,0 g/mol, Feststoff
MALDI-TOF (m/z) = 881 [MNa⁺]
Elementaranalyse: berechnet: C 71,31 Gew.-%, H 6,34 Gew.-%
gefunden: C 69,95 Gew.-%, H 6,21 Gew.-% Phasenverhalten: K 123 I

Eine Lösung aus 4-Hexyloxybenzoyl-4'-oxybenzoesäure (2 mmol), N,N'-Dicyclohexylcarbodiimid (2,2 mmol), 2,4-O-Benzyliden-D-threitol (1,1 mmol) und dem Katalysator 4-N,N-Dimethylaminopyridin (4-DMAP; 0,1 mmol) in Dichlormethan (5 ml) wird bei Raumtemperatur bis zur vollständigen Umsetzung gerührt. Der gebildete N,N'-Dicyclohexylharnstoff wird abfiltriert und das Lösungsmittel im Vakuum entfernt. Der Rückstand wird säulenchromatographisch gereinigt (Laufmittel: Petrolether 60/70 : Ethylacetat 8:1) und aus Ethanol um-kristallisiert. Ausbeute: 0,48 g (56% d.Th.)

¹H-NMR (400 MHz, CDCl₃): δ = [8,23 (d, 2H), 8,13 (d, 2H), 8,12 (d, 2H), 8,09 (d, 2H), H_{Ar}-2, H_{Ar}-6, H_{Ar}-2', H_{Ar}-6', H_{Ar}-2'', H_{Ar}-6'', H_{Ar}-2''', H_{Ar}-6'''], 7,58 (mc, 2H, H_{Bz}-2, H_{Bz}-6), 7,41 (mc, 3H, H_{Bz}-3, H_{Bz}-4, H_{Bz}-5), [7,32 (d, 2H), 7,28 (d, 2H), H_{Ar}-3, H_{Ar}-5, H_{Ar}-3'', H_{Ar}-5''], 6,97 (mc, 4H, H_{Ar}-3', H_{Ar}-5',H_{Ar}-3''', H_{Ar}-5'''), 5,73 (s, 1H, OCHO), 5,20 (ddd, 1H, H-5), 4,66 (dd, 1H, C***H***_{***A***}H_{B}OOC), 4,58 (ddd, 1H, H-4), 4,53 (dd, 1H, CH_{A}***H***_{***B***}OOC), 4,51 (dd, 1H, H-6a), 4,26 (dd, 1H, H-6b), 4,04 (t, 4H, OC***H***_{***2***}C₅H₁₁), 1,81 (mc, 4H, OCH₂C***H***_{***2***}C₄H₉), 1,53-1,30 (m, 12H, 6xCH₂), 0,92 (t, 6H, CH₃).

³J_{Ar} = 9,1, ³J_{H-4, H-5} = 1,5, ³J_{H-4, Ha} = 6,2, ³J_{H-4, Hb} = 5,6, ³J_{H-5,} _{H-6a} = 1,5, ³J_{H-5, H-6b} = 1,5, ²J_{Ha, Hb} = 10,7, ²J_{H-6a, H-6b} = 13,2, ³J_{OCH2} = 6,6 Hz.

¹³C-NMR (100 MHz, CDCl₃): δ = 165,53, 165,39, 164,32, 164,31, 163,76 (4xCOO, C_{Ar}-4', C_{Ar}-4'''), 155,25, 155,06 (C_{Ar}-4, C_{Ar}-4''), 137,57 (C_{Bz}-1), 132,41, 132,38 (C_{Ar}-2', C_{Ar}-6', C_{Ar}-2''', C_{Ar}-6'''), 131,55, 131,35 (C_{Ar}-2, C_{Ar}-6, C_{Ar}-2'', C_{Ar}-6''), 129,26 (C_{Bz}-4), 128,39 (C_{Bz}-3, C_{Bz}-5), 126,98, 126,97 (C_{Ar}-1, C_{Ar}-1''), 126,22 (C_{Bz}-2, C_{Bz}-6), 122,03, 121,93 (C_{Ar}-3, C_{Ar}-5, C_{Ar}-3'', C_{Ar}-5''), 121,02, 121,00 (C_{Ar}-1', C_{Ar}-1'''), 114,38 (C_{Ar}-3', C_{Ar}-5', C_{Ar}-3''', C_{Ar}-5'''), 101,39 (C-2), 75,61 (C-4), 69,37 (C-6), 68,36 (OCH₂), 65,87 (C-5), 63,40 (***C***H₂OOC), 31,54, 29,04, 25,64, 22,58 (CH₂), 14,02 (CH₃).

### Beispiel 4: Herstellung von (2S,4R,5R)-4,5-Di-O-(4-(Acryloxybutyloxycarbonlyoxy)benzoyl)-2-phenyl-1,3-dioxan

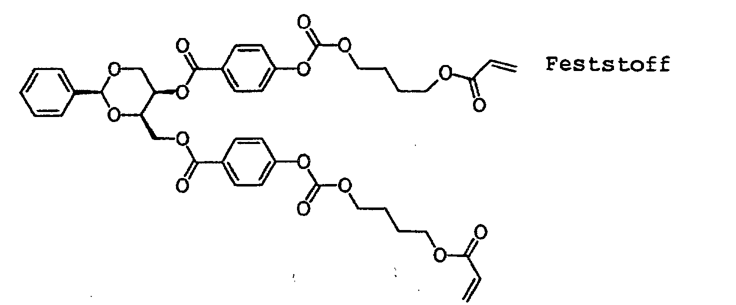

Eine Lösung aus 4-(Acryloxybutyloxycarbonyloxy)-benzoesäure (2 mmol), N,N'-Dicyclohexylcarbodlimid (2,2 mmol), 2,4-O-Benzyliden-D-threitol (1,1 mmol; s. Beispiel 1c) und dem Katalysator 4-DMAP (0,1 mmol) in Dichlormethan (5 ml) wird bei Raumtemperatur bis zur vollständigen Umsetzung gerührt, der gebildete N,N'-Dicyclohexylharnstoff abfiltriert und das Lösungsmittel im Vakuum entfernt. Schließlich wird der Rückstand säulenchromatographisch gereinigt und aus Ethanol umkristallisiert.

### Beispiel 7: Herstellung vernetzter cholesterischer Filme

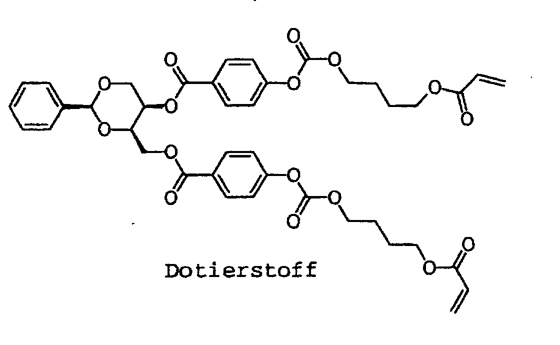

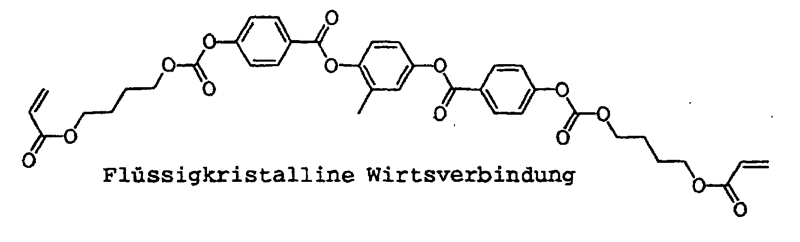

Es wird eine cholesterische Mischung zubereitet, welche die gezeigten Verbindungen als chiralen Dotierstoff bzw. flüssigkristalline Wirtsverbindung enthält. Die unverdünnte cholesterische Mischung enthält 94,2 Gew.-% der nematischen Wirtsverbindung, 5,8 Gew.-% des chiralen Dotierstoffs und als Photoinitiator 2 Gew.-%, bezogen auf die cholesterische Mischung, 1-Hydroxycyclohexylphenylketon, das unter der Bezeichnung Irgacure 184 (Fa. Ciba) vertrieben wird. Die Mischung zeigt ein Maximum der Reflektion bei einer Wellenlänge von λₘₐₓ = 352 nm.

Zur Filmherstellung wird diese Mischung in Methylethylketon gelöst und als dünne Schicht auf eine Polyethylenterephthalatfolie aufgerakelt. Die Nassfilmdicke der Schicht beträgt hierbei etwa 3 µm. Nach dem Abdampfen des Lösungsmittels bei 75°C wird die Schicht durch UV-Bestrahlung ausgehärtet. Die erhaltene polymerisierte bzw. vernetzte cholesterische Schicht lässt sich mechanisch vom Träger ablösen. Sofern gewünscht können die so erhaltenen Flitter mittels gängiger Mahlmethoden auf die gewünschte Größe zerkleinert und beispielsweise als Pigmente verwendet werden.

## Patentansprüche

1. Chirale Verbindungen der allgemeinen Formel I 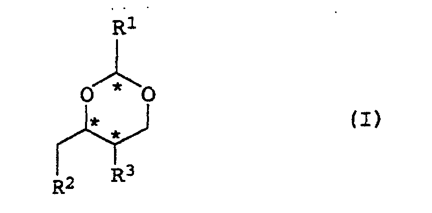 und deren Diastereomere, in welchen bedeuten
R¹ eine Gruppierung [P-Y¹-(A¹)ₘ-Y²-]_{q}M-Y³-(A²)ₙ-Y⁴- und
R² und R³ unabhängig voneinander und unabhängig von R¹ Gruppierungen [P-Y¹-(A¹)ₘ-Y²-]_{q}M-Y³-(A²)ₙ-Y^{4'}-
mit
A¹, A² Spacer mit ein bis 30 Kohlenstoffatomen,
M mesogene Gruppe,
Y¹, Y², Y³, Y⁴ chemische Einfachbindung, -O-, -S-, -CO-, - CO-O-, -O-CO-, -CO-N(R)-, -(R)N-CO-, -O-CO-O-, - O-CO-N(R)-, -(R)N-CO-O- oder -(R)N-CO-N(R)-,
Y⁴' -O-, -O-CO-, -O-CO-O- oder -O-CO-N(R)-,
R Wasserstoff oder C₁-C₄-Alkyl,
P Wasserstoff, C₁-C₁₂-Alkyl, eine polymerisierbare oder zur Polymerisation geeignete Gruppe oder ein Rest, welcher eine polymerisierbare oder zur Polymerisation geeignete Gruppe trägt,
m, n Werte von 0 oder 1 und
q Werte von 1, 2 oder 3,
wobei die Reste A¹, A², Y¹, Y², Y³, Y⁴, Y⁴', M und P und die Indices m, n und q der Reste R¹ bis R³ gleich oder verschieden sein können und die Reste R² und R³ über das Sauerstoff atom des Restes Y⁴' an das 1,3-Dioxangerüst angebunden sind, mit der Maßgabe, dass für den Fall, dass einer oder beide der Indices m und n gleich 0 sind, mindestens einer der jeweils von A benachbarten Reste Y eine chemische Bindung bedeutet.

2. Verbindungen nach Anspruch 1, in welchen die mesogene Gruppe M der Formel Ia
(-T-Y⁵)ᵣ-T- Ia
entspricht, worin die Variablen bedeuten
T zweiwertige gesättigte oder ungesättigte carbo- oder heterocyclische Reste
Y⁵ -chemische Einfachbindung, -O-, -S-, -CO-, -CO-O-, -O-CO-, -CO-N(R)-, -(R)N-CO-, -O-CO-O-, -O-CO-N(R)-, -(R)N-CO-O- oder -(R)N-CO-N(R)- und
r Werte von 0, 1, 2 oder 3, wobei für r > 0 sowohl die Reste T als auch die Gruppen Y⁵ untereinander gleich oder verschieden sein können.

3. Verbindungen nach Anspruch 2, in welchen in der mesogenen Gruppe der Formel Ia für den Rest R¹ der Index r den Wert 0 und für die Reste R² und R³ unabhängig voneinander die Werte 0 oder 1 annimmt.

4. Verbindungen nach Anspruch 2 oder 3, in welchen T ausgewählt ist aus der Gruppe bestehend aus 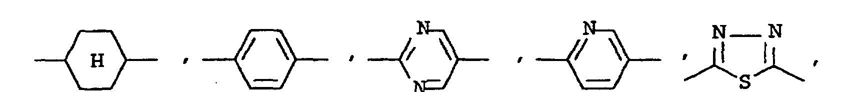 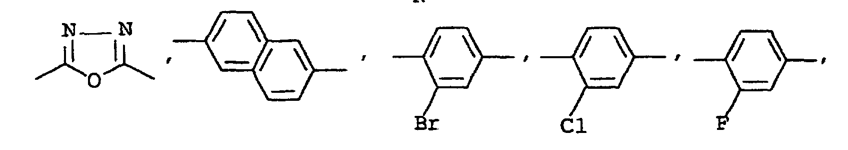 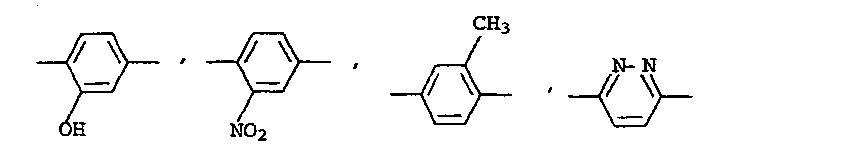 und

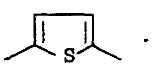

5. Verbindungen nach einem oder mehreren der Ansprüche 1 bis 4, in welchen im Rest R¹ die Einheit P-Y¹-(A¹)ₘ-Y²- Wasserstoff (P Wasserstoff, Y¹ und Y² chemische Einfachbindung und m gleich 0) bedeutet und in mindestens einer der Einheiten P-Y¹-(A¹)ₘ-Y²- der beiden Reste R² und R³ m verschieden von 0 ist.

6. Verbindungen nach einem oder mehreren der Ansprüche 1 bis 5, in welchen q in den beiden Resten R² und R³ den Wert 1 annimmt.

7. Verwendung von Verbindungen nach einem oder mehreren der Ansprüche 1 bis 6 als chirale Dotierstoffe für flüssigkristalline Systeme.

8. Flüssigkristalline Zusammensetzungen, enthaltend mindestens eine chirale Verbindung der allgemeinen Formel I gemäß einem oder mehreren der Ansprüche 1 bis 6.

9. Polymerisierbare flüssigkristalline Zusammensetzungen, enthaltend mindestens eine chirale Verbindung der allgemeinen Formel I gemäß einem oder mehreren der Ansprüche 1 bis 6.

10. Verwendung von Zusammensetzungen gemäß Anspruch 8 oder 9 für die Herstellung optischer Bauelemente.

11. Optische Bauelemente, welche unter Verwendung von Zusammensetzungen gemäß Anspruch 8 oder 9 hergestellt worden sind.

12. Verwendung von Zusammensetzungen gemäß Anspruch 9 zum Bedrukken oder Beschichten von Substraten, zur Herstellung von Dispersionen und Emulsionen, zur Herstellung von Filmen oder zur Herstellung von Pigmenten.

13. Bedruckte oder beschichtete Substrate, Dispersionen und Emulsionen, Filme oder Pigmente, welche unter Verwendung von Zusammensetzungen gemäß Anspruch 9 hergestellt worden sind.

## Claims

1. A chiral compound of the general formula I 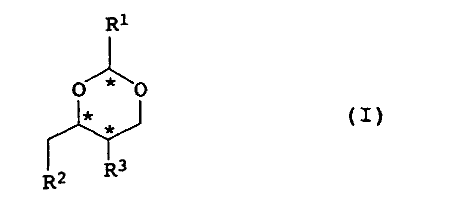 or a diastereomer thereof, where
R¹ is [P-Y¹-(A¹)ₘ-Y²-]_{q}M-Y³-(A²)ₙ-Y⁴-, and
R² and R³ are each, independently of one another and independently of R¹, [P-Y¹-(A¹)ₘ-Y²-]_{q}M-Y³-(A²)ₙ-Y⁴'-,
where
A¹ and A² are each a spacer having from one to 30 carbon atoms,
M is a mesogenic group,
Y¹, Y², Y³ and Y⁴ are each a chemical single bond, -O-, -S-, -CO-, -CO-O-, -O-CO-, -CO-N(R)-, -(R)N-CO-, -O-CO-O-, - O-CO-N(R)-, -(R)N-CO-O- or -(R)N-CO-N(R)-,
Y⁴' is -O-, -O-CO-, -O-CO-O- or -O-CO-N(R)-,
R is hydrogen or C₁-C₄-alkyl,
P is hydrogen, C₁-C₁₂-alkyl, a polymerizable group or a group suitable for polymerization or a radical having a polymerizable group or a group suitable for polymerization,
m and n are each 0 or 1, and
q is 1, 2 or 3,
where A¹, A², Y¹, Y², Y³, Y⁴, Y⁴', M and P and the indices m, n and q of R¹ to R³ can be identical or different, and R² and R³ are attached to the 1,3-dioxane skeleton via the oxygen atom of Y⁴', with the proviso that at least one of the radicals Y, in each case adjacent to A, is a chemical bond if one or both of the indices m and n is/are 0.

2. A compound as claimed in claim 1, where the mesogenic group M has the formula Ia
(-T-Y⁵)ᵣ-T- Ia
where
T at each occurrence is a divalent, saturated or unsaturated carbocyclic or heterocyclic radical,
Y⁵ at each occurrence is a chemical single bond, -O-, -S-, -CO-, -CO-O-, -O-CO-, -CO-N(R)-, -(R)N-CO-, -O-CO-O-, -O-CO-N(R)-, -(R)N-CO-O- or -(R)N-CO-N(R)- and
r is 0, 1, 2 or 3, where, if r > 0, both T and Y⁵ can be identical or different at each occurrence.

3. A compound as claimed in claim 2, where, in the mesogenic group of the formula Ia, the index r is 0 for R¹ and the index r, independently at each occurrence, is 0 or 1 for R² and R³.

4. A compound as claimed in claim 2 or 3, where T is selected from the group consisting of 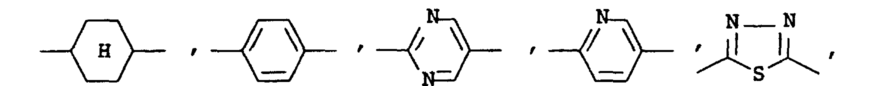 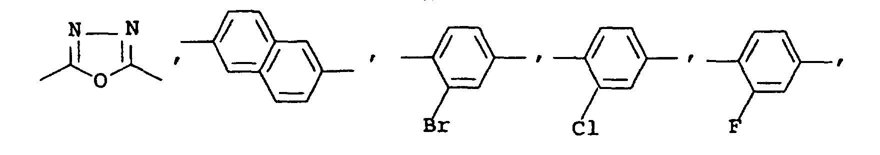 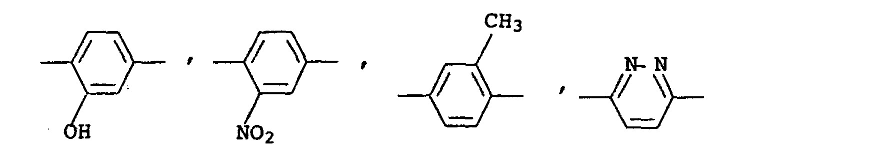 and

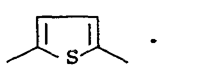

5. A compound as claimed in one or more of claims 1 to 4, where, in R¹, P-Y¹-(A¹)ₘ-Y²- is hydrogen (P is hydrogen, Y¹ and Y² are each a chemical single bond and m is 0), and m is not 0 in at least one P-Y¹-(A¹)ₘ-Y²- of R² and R³.

6. A compound as claimed in one or more of claims 1 to 5, where q is 1 in R² and R³.

7. The use of compounds as claimed in one or more of claims 1 to 6 as chiral dopants for liquid-crystalline systems.

8. A liquid-crystalline composition comprising at least one chiral compound of the general formula I as claimed in one or more of claims 1 to 6.

9. A polymerizable liquid-crystalline composition comprising at least one chiral compound of the general formula I as claimed in one or more of claims 1 to 6.

10. The use of compositions as claimed in claim 8 or 9 for producing optical components.

11. An optical component which has been produced using a composition as claimed in claim 8 or 9.

12. The use of compositions as claimed in claim 9 for printing or coating a substrate, for preparing a dispersion or emulsion, for producing a film or for preparing a pigment.

13. A printed or coated substrate, dispersion or emulsion, film or pigment which has been produced using a composition as claimed in claim 9.

## Revendications

1. Composés chiraux de formule générale I 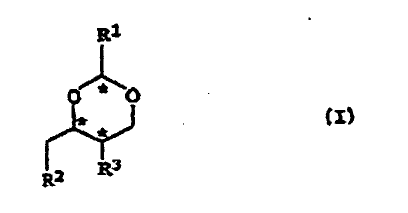 et leurs diélastomères, dans lesquels
R¹ représente un groupement [P-Y¹-(A¹)ₘ-Y²-]_{q}M-Y³-(A²)ₙ-Y⁴- et
R² et R³ représentent, indépendamment l'un de l'autre et indépendamment de R¹, des groupements [P-Y¹-(A¹)ₘ-Y²-]_{q}M-Y³-(A²)ₙ-Y⁴'-
dans lesquels
A¹, A² représentent des espaceurs possédant un à 30 atomes de C,
M représente un groupe mésogène,
Y¹, Y², Y³, Y⁴ représentent une liaison chimique simple, -O-, -S-, -CO-, -CO-O-, -O-CO-, -CO-N(R)-, - (R)N-CO-, -O-CO-O-, -O-CO-N(R)-, -(R)N-N-CO-O- ou -(R)N-CO-N(R)-,
Y⁴' représente -O-, -O-CO-, -O-CO-O-, ou - O-CO-N (R) -,
R représente un atome d'hydrogène ou un groupe alkyle en C₁ à C₄,
P représente un atome d'hydrogène, un groupe alkyle en C₁ à C₁₂, un groupe approprié à la polymérisation ou un radical possédant un groupe polymérisable ou approprié à la polymérisation,
m, n valent 0 ou 1 et
q vaut 1, 2 ou 3,
les groupes A¹, A², Y¹, Y², Y³, Y⁴, Y⁴', M et P et les indices m, n et q des groupes R¹ à R³ pouvant être identiques ou différents et les groupes R² et R³ étant liés par l'intermédiaire de l'atome d'oxygène du groupe Y⁴' au squelette de 1,3-dioxane, avec pour condition que dans le cas où l'un des indices ou les deux indices m ou n valent 0, au moins un des groupes Y vicinaux de A représente une liaison chimique.

2. Composés selon la revendication 1, dans lesquels le groupe mésogène M correspond à la formule Ia
(-T-Y⁵)ᵣ-T- Ia,
dans laquelle les variables ont la signification suivante :
T représente des groupes carbocycliques ou hétérocycliques saturés ou insaturés bivalents
Y⁵ représente une liaison chimique simple, -O-, -S-, -CO-, -CO-O-, -O-CO-, -CO-N(R)-, -(R)N-CO-, -O-CO-O-, -O-CO-N(R)-, -(R)N-CO-O ou -(R)N-CO-N(R)-,
et
r vaut 0, 1, 2 ou 3, les groupes T ainsi que les groupes Y⁵ pouvant être identiques ou différents si r > 0.

3. Composés selon la revendication 2, dans lesquels l'indice r prend la valeur 0 pour le groupe R¹ dans le groupe mésogène de formule Ia et prend les valeurs 0 ou 1 pour les groupes R² et R³ indépendamment l'un de l'autre.

4. Composés selon la revendication 2 ou 3,
dans lesquels T est choisi dans le groupe formé par 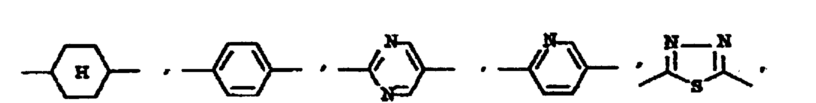 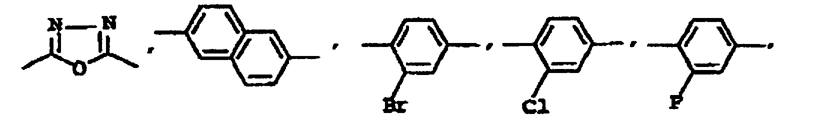 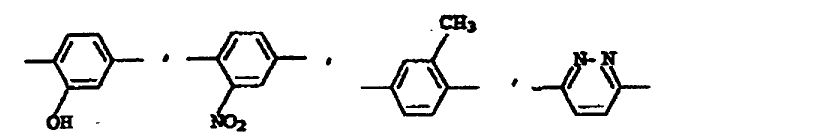 et

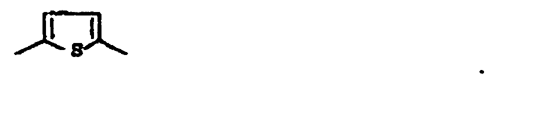

5. Composés selon l'une quelconque ou plusieurs des revendications 1 à 4, dans lesquels le groupe R¹ représente le motif P-Y¹-(A¹)ₘ-Y²-hydrogène (P représente un atome d'hydrogène, Y¹ et Y² représentent une liaison chimique simple et m vaut 0) et dans au moins l'un des motifs P-Y¹-(A¹)ₘ-Y²- des deux groupes R² et R³, m est différent de 0.

6. Composés selon l'une quelconque ou plusieurs des revendications 1 à 5, dans lesquels q vaut 1 dans les deux groupes R² et R³.

7. Utilisation des composés selon l'une quelconque ou plusieurs des revendications 1 à 6 en tant qu'agents de dopage chiraux pour des systèmes à cristaux liquides.

8. Compositions à cristaux liquides contenant au moins un composé chiral de formule générale I selon l'une quelconque ou plusieurs des revendications 1 à 6.

9. Compositions à cristaux liquides polymérisables contenant au moins un composé chiral de formule générale I selon l'une quelconque ou plusieurs des revendications 1 à 6.

10. Utilisation de compositions selon la revendication 8 ou 9 pour la fabrication de composants optiques.

11. Composants optiques qui ont été fabriqués en utilisant des compositions selon la revendication 8 ou 9.

12. Utilisation de compositions selon la revendication 9 pour l'impression ou le revêtement de substrats, pour la préparation de dispersions et d'émulsions, pour la fabrication de films ou pour la préparation de pigments.

13. Substrats imprimés ou revêtus, dispersions et émulsions, films et pigments qui ont été fabriqués en utilisant des compositions selon la revendication 9.
